# EUROPEAN PATENT SPECIFICATION

(11) **EP 0 412 941 B1**
(45) Date of publication and mention of the grant of the patent: **15.05.1996**
(21) Application number: 90810594.3
(22) Date of filing: 07.08.1990
(51) Int. Cl.: A61K 31/685

(54) **Phospholipids for the treatment of multiple sclerosis**
Phospholipide zur Behandlung der multiplen Sklerose
Phospholipides pour le traitement de la sclérose multiple

(30) Priority: 10.08.1989 US 392187; 18.10.1989 US 423323; 07.03.1990 US 489578
(43) Date of publication of application: 13.02.1991
(73) Proprietor: SANDOZ LTD., CH-4002 Basel (CH); SANDOZ-PATENT-GMBH, D-79539 Lörrach (DE); SANDOZ-ERFINDUNGEN Verwaltungsgesellschaft m.b.H., A-1235 Wien (AT)
(72) Inventor: Houlihan, William Joseph, Mountain Lakes, N.J. 07046 (US)

(56) References cited:
- EP-A- 0 225 608
- WO-A-87/01257
- US-A- 4 749 696
- JOURNAL OF NEUROLOGY, vol. 207, no. 4, 1974, pages 319-326, Springer-Verlag; H. WOELK et al.: "Zur Aktivität der Phospholipase A2 gegenüber verschiedenen 1- Alk-1'-enyl-2-acyl- und 1-Alkyl-2-acyl-Verbindungen während der multiplen Sklerose
- BIOCHIMICA ET BIOPHYSICA ACTA, vol. 820, no. 2, 1985, pages 319-323, Elsevier Science Publishers B.V. (Biomedical Division); W.K. SUREWICZ et al.: "Aliphatic aldehydes promote myelin basic protein-induced fusion of phospholipid vesicles"

## Description

The present invention relates to the use of certain heterocyclic, thioether, keto-ester and alkyl phospholipids in the treatment of multiple sclerosis.

Multiple sclerosis, a crippling nerve disorder characterized by disseminated patches of demyelination in the brain and spinal cord, has occupied the attention of research organizations for many years without, unfortunately, any appreciable success. Although ACTH (adrenocorticotropic hormone) or prednisone appears to hasten recovery in acute attacks, especially when administered early in the episode, there is no specific therapy, even today, as spontaneous remissions make any treatment difficult to evaluate.

USP 4,778,788 discloses certain lysolecithin analogs useful in treating multiple sclerosis.

It has now been found that, surprisingly, certain heterocyclic, thioether, keto-ester and alkyl phospholipids are useful in treating multiple sclerosis. Accordingly, the present invention provides a method of treating multiple sclerosis by administering an effective amount of a compound of formula I: 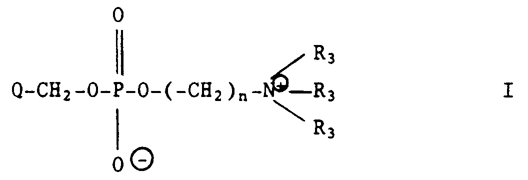
in which n is an integer from 2 to 6;
each R₃, independently, is methyl or ethyl;
and Q is a group of formula II or a group of formula III. 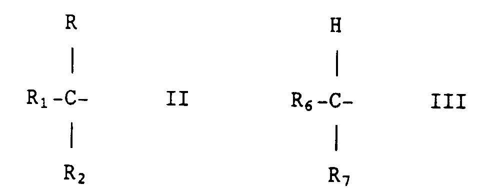
In the group of formula II,
- R is: hydrogen or together with R₁ forms a group -YCH₂CH₂CH₂- where Y is -O- or -S-;
- R₁ and R₂,: when R is hydrogen, together form a group 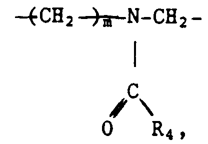 where m is 2 to 4 and R₄ is n-C(14-20) alkyl or n-C(14-20) alkoxy,
or a group 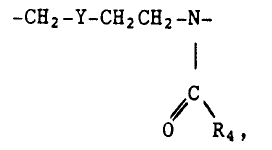 where R₄ and Y are defined above,
or a group 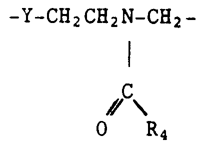 where R₄ and Y are defined above,
or, when R and R₁ form a group -YCH₂CH₂CH₂-, R₂ is a group -CH₂OR₅ where R₅ is n-C(14-20) alkyl.

In the group of formula III,
R₆ is hydrogen and R₇ is n-C(12-18) alkyl, or
R₆ is -CH₂SR₅ and R₇ is -CH₂OR₃, or
R₆ is -CH₂OR₅ and R₇ is -OCOCH₂COCH₃;
where R₃ and R₅ are defined above.

Preferably n is n' where n' is 2,3 or 4, more preferably 2. R₃ is preferably methyl. R₄ is preferably R₄' where R₄' is n-C(14-18)alkyl or n-C(14-18)alkoxy; more preferably it is n-C(14-18)alkoxy, particularly n-C₁₈H₃₇O-. R₅ is preferably R₅' where R₅' is n-C(14-18)alkyl, particularly n-C₁₆ or C₁₈ alkyl.

A class of compounds of formula I in which Q is a group of formula II found useful in the practice of this invention is the heterocyclic phospholipids of formula V: 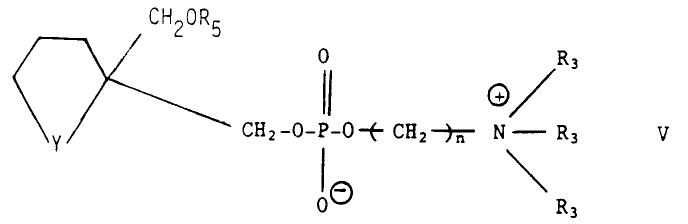
where Y, R₃, R₅, and n are as defined above.

The preferred compounds of formula V are compounds of formula V′ 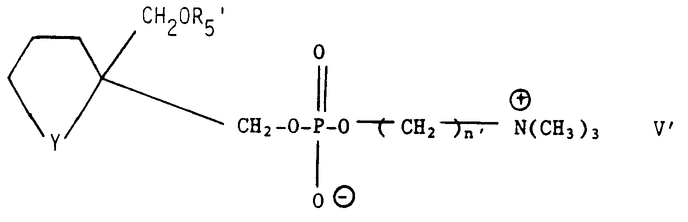
where n′, Y and R₅′ are as defined above.

The most preferred compound of formula V is 2-[[2-octadecyloxymethyltetrahydro-2-furanylmethoxy)-hydroxy-phosphinyloxy]-N,N,N,-trimethylethanaminium hydroxide inner salt-4-oxide having the formula

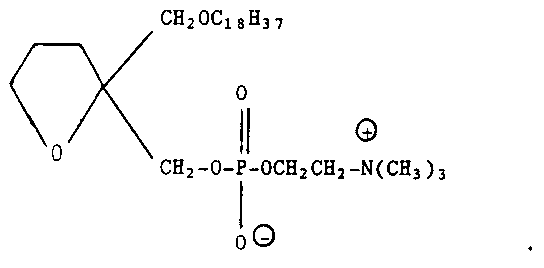

The compounds of formula V and their use as anti-tumor agents are disclosed in USP 4,673,672, and all of the compounds of formula V may be prepared according to the processes described therein.

The compounds of formula V contain an asymmetric carbon atom. It should be understood, therefore, that the compounds may exist in racemic or enantiomeric form, and all forms are included within the practice of this invention. Enantiomeric forms may be recovered in conventional manner, e.g., by resolution of end or intermediate products or by employing optically active starting materials.

As indicated above, all of the compounds of formula V are useful in treating multiple sclerosis, as indicated by the following test methods:

### Experimentally Induced Allergic Encephalomyelitis (EAE) in the Rat [Levine et al., AM. J. PATH. 47 (1965) 61; McFarlin et al, J. IMMUNOL. 113 (1974) 712; Borel, TRANSPLANT & CLIN. IMMUNOL. 13 (1981) 3].

Male Wistar rats are injected in the hind paws with a mixture of bovine spinal cord and complete Freund's adjuvant. Symptoms of the disease (paralysis of the tail and both hind legs) usually develop within 16 days. The number of diseased animals as well as the time of onset of the disease are recorded.

On administration at dosages of from about 5 to about 50mg/kg/day p.o., 5 days a week, commencing on the day of sensitization and continuing for 3 weeks, compounds of formula I inhibit disease onset in the above test model.

### Established Experimental Allergic Encephalomyelitis (EEAE):

Testing is carried out analogously to that described above with the exception that the administration of the test compound commences on day 8 to day 9 after sensitization (i.e., immediately prior to appearance of disease symptoms) at dosages of from 5 to 50 mg/kg/day either daily or every second day and continuing for 2 weeks. During the testing period, the animals are examined daily for symptoms of the disease and scored as in the above test method.

On administration of compounds of formula I at the above dosage rates, a substantial reduction of appearance of EAE disease symptoms is observed over the test period in comparison with appearance in control groups receiving placebo.

The precise dosage of a compound of formula I, particularly a compound of formula V to be employed in treating multiple sclerosis depends upon several factors including the host, the nature and the severity of the condition being treated, the mode of administration and the particular compound employed. However, in general, satisfactory inhibition of the symptoms of multiple sclerosis is achieved when a compound of formula I, is administered orally at a daily dosage of 0.5-30 mg/kg body weight, preferably 1-20 mg/kg or, for most larger primates, at a total daily dosage of 35-600 mg. A preferred total daily dosage for most larger primates is 150 to 300 mg.

Usually, a small dosage is administered initially and the dosage is gradually increased until the optimal dosage for the host under treatment is determined. The upper limit of dosage is that imposed by side effects, and can be determined by trials for the host being treated, including humans.

As indicated above, a preferred total daily dosage for most larger primates, e.g., humans, is 150 to 300 mg. However, it should be understood that when a clear improvement in the symptoms of multiple sclerosis is observed upon daily administration of between 150 and 300 mg of a compound of formula I, the dosage regimen can be decreased to between 150 and 300 mg of a compound of formula I, every second day.

The compounds of formula I may be combined with one or more pharmaceutically acceptable carriers and, optionally, one or more conventional pharmaceutical adjuvants and administered orally in the form of tablets, dispersible powders, granules, capsules, elixirs, suspensions and the like. The compositions may be prepared by conventional means. Advantageously, the compounds may be dissolved in a small quantity of a suitable solvent, for example ethanol, and diluted with milk for drinking.

The compounds of formula I may be formulated into such pharmaceutical compositions containing an amount of the active substance that is effective in treating multiple sclerosis, such compositions in unit dosage form and such compositions comprising a solid pharmaceutically acceptable carrier.

Tablets and capsules containing the ingredients indicated below may be prepared by conventional techniques and are useful in treating multiple sclerosis when administered once a day.

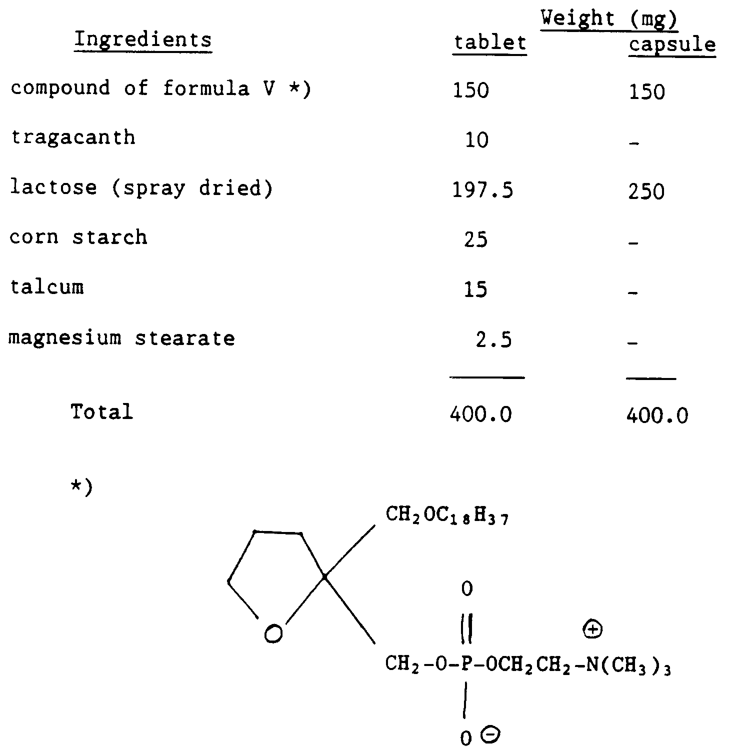

The present invention additionally provides:
1. The use of a compound of formula I for the treatment of multiple sclerosis.
2. The use of a compound of formula I for the preparation of a medicament for the treatment of multiple sclerosis.
3. A compound of formula I for the treatment of multiple sclerosis.
4. A pharmaceutical composition for the treatment of multiple sclerosis comprising a compound of formula I in association with a pharmaceutically acceptable diluent or carrier.

## Claims

1. The use of a compound of formula V 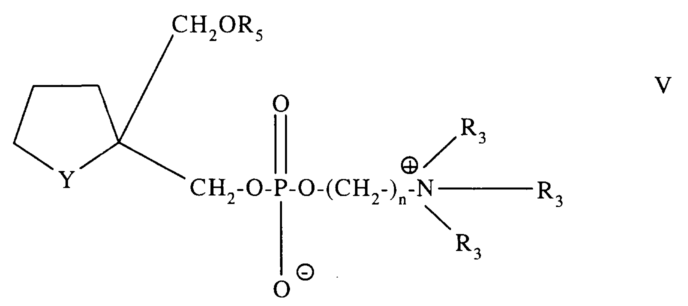 where
Y is -O- or -S-;
R₃ is, independently, methyl or ethyl;
R₅ is n-C(₁₄₋₂₀)alkyl; and
n is an integer from 2 to 6;
for the preparation of a medicament for the treatment of multiple sclerosis.

2. The use according to claim 1 in which the compound of formula V is a compound of formula V' 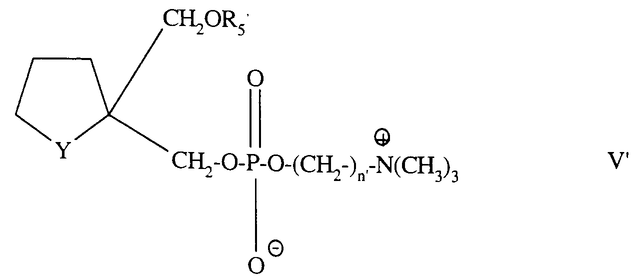 where R₅' is n-C(₁₄₋₁₈)alkyl, n' is 2, 3 or 4 and Y is as defined in Claim 1.

3. The use according to claim 2 where the compound has the formula

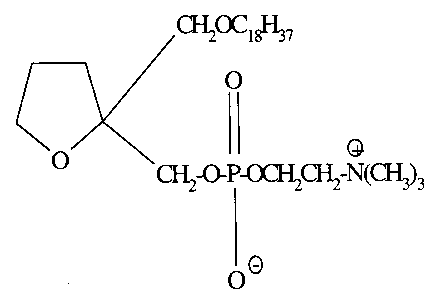

4. The use according to any one of claims 1-3 in which the daily dosage is from 150-300 mg p.o.

## Patentansprüche

1. Die Verwendung einer Verbindung der Formel V 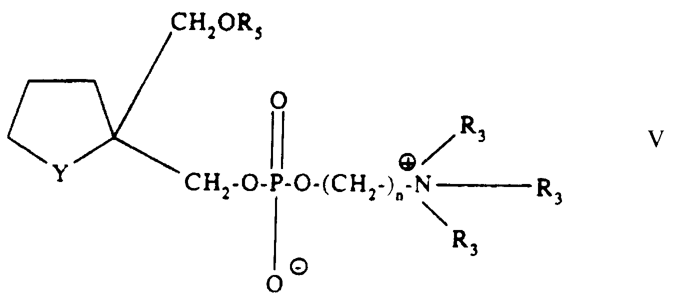 worin
Y -O- oder -S- bedeutet;
R₃ unabhängig für Methyl oder Ethyl steht;
R₅ n-C(₁₄₋₂₀)Alkyl bedeutet; und
n für eine ganze Zahl von 2 bis 6 steht;
zur Herstellung eines Medikamentes zur Behandlung der Multiplen Sklerose.

2. Die Verwendung gemäss Patentanspruch 1, worin die Verbindung der Formel V eine Verbindung der Formel V' 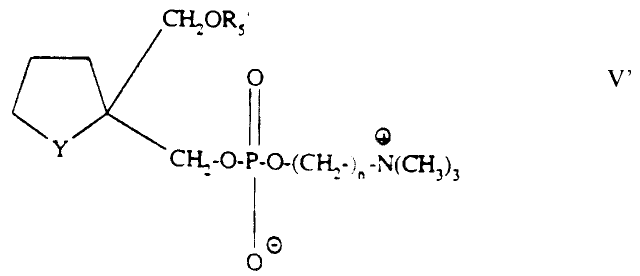 ist, worin R₅' für n-C(₁₄₋₁₈)Alkyl, n' für 2, 3 oder 4 stehen und Y in Patentanspruch 1 definiert ist.

3. Die Verwendung gemäss Patentanspruch 2, worin die Verbindung die Formel 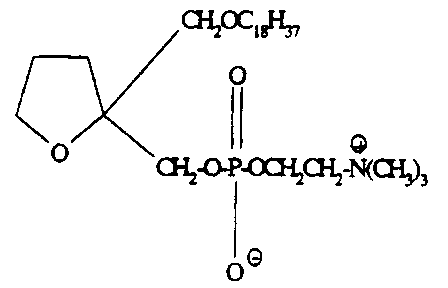 besitzt.

4. Die Verwendung gemäss einem der Patentansprüche 1-3, worin die tägliche Dosis von 150-300 mg p.o.beträgt.

## Revendications

1. L'utilisation d'un composé de formule V 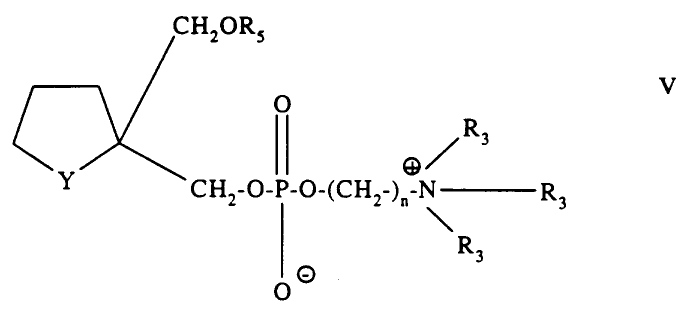 dans laquelle
Y signifie -O- ou -S-,
R₃ signifie indépendamment un groupe méthyle ou éthyle,
R₅ signifie un groupe n-C-alkyle en C₁₄-C₂₀, et
n signifie un nombre entier de 2 à 6,
pour la préparation d'un médicament pour le traitement de la sclérose multiple.

2. L'utilisation selon la revendication 1, dans laquelle le composé de formule V est un composé de formule V' 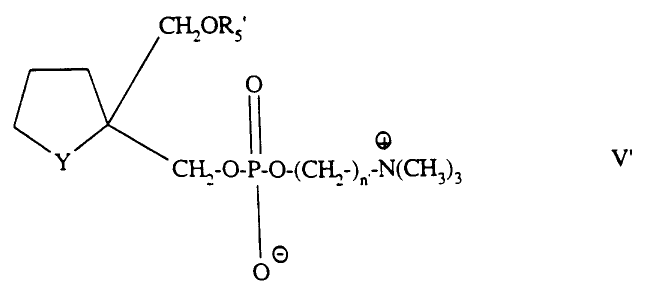 dans laquelle R₅' signifie un groupe n-C-alkyle en C₁₄-C₁₈, n' signifie 2, 3 ou 4 et Y est tel que défini à la revendication 1.

3. L'utilisation selon la revendication 2 dans laquelle le composé répond à la formule

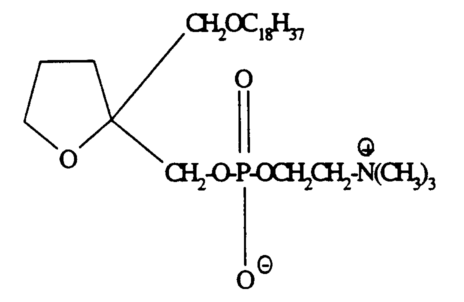

4. L'utilisation selon l'une quelconque des revendications 1 à 3 dans laquelle la dose quotidienne est comprise entre 150 et 300 mg par voie orale.
